# EUROPÄISCHE PATENTSCHRIFT

(11) **EP 0 900 063 B1**
(45) Veröffentlichungstag und Bekanntmachung des Hinweises auf die Patenterteilung: **06.04.2005**
(21) Anmeldenummer: 98909471.9
(22) Anmeldetag: 17.02.1998
(51) Int. Cl.: A61F 2/06

(54) **STENT**
STENT
ENDOPROTHESE

(30) Priorität: 17.02.1997 DE 29702671 U
(43) Veröffentlichungstag der Anmeldung: 10.03.1999
(60) Teilanmeldung: 04030672.2
(73) Patentinhaber: Abbott Laboratories Vascular Enterprises Limited, Dublin 2 (IE)
(72) Erfinder: VON OEPEN, Randolf, D-72145 Hirrlingen (DE)
(74) Vertreter: Schmitz, Hans-Werner, Dipl.-Ing.
(86) Internationale Anmeldenummer: PCT/EP1998/000884
(87) Internationale Veröffentlichungsnummer: WO 1998/035634

(56) Entgegenhaltungen:
- WO-A-96/03092
- WO-A-97/32543
- DE-U- 29 608 037
- DE-U- 29 615 969
- DE-U- 29 708 879
- DE-U- 29 716 476
- US-A- 5 697 971

## Beschreibung

Die Erfindung betrifft einen Stent nach dem Oberbegriff des Anspruchs 1.

Unter einem Stent versteht man eine medizinisch-technische Vorrichtung, die an die Stelle von Verengungen von Körpergef äßen oder Körperhöhlungen eingeführt wird und dort im aufgeweiteten Zustand die Verengung aufweitet und im aufgeweiteten Zustand hält. Hierzu muß ein Stent im nicht-expandierten Zustand äußerst flexibel sein, damit er beim Einführen in die Körpergefäße deren windungen problemlos folgen kann. Ferner muß der Stent im aufgeweiteten Zustand ausreichend stabil sein, um das gewünschte Aufweitungsmaß aufrechterhalten zu können.

Obwohl der gattungsgemäße Stent (DE 296 08 037.1) diese Anforderungen bereits zufriedenstellend erfüllt, ist grundsätzlich eine weitere Verbesserung der Stenteigenschaften wünschenswert. Weitere gattungsgemässe Stents sind aus der WO 06 03092 A und der DE 296 15 969 bekannt. Einen Stand der Tecknik gemäss Artikel 53(3) und (4) EPÜ stellt die EP O 821 921 dar.

Es ist daher Aufgabe der vorliegenden Erfindung, einen Stent der im Oberbegriff des Anspruchs 1 angegebenen Gattung zu schaffen, dessen Eigenschaften hinsichtlich Flexibilität im nicht-expandierten Zustand und Formstabilität im expandierten Zustand weiter verbessert sind.

Die Lösung dieser Aufgabe erfolgt durch die Merkmale des Anspruches 1.

Der erfindungsgemäße Stent zeichnet sich durch eine Verbesserung der an sich gegenläufigen Eigenschaften hoher Flexibilität im nicht-expandierten Zustand und hoher Formstabilität im expandierten Zustand aus.

Mit dem erfindungsgemäßen Stent wird eine Konstruktion geschaffen, deren Stegstruktur eine Vielzahl von aneinander angrenzenden Zellen aufweist. Die Stegstruktur kann bei unterschiedlichen Ausführungsformen aus unterschiedlich aufgebauten Stegen bzw. Schenkeln bestehen, die Zellen umgrenzen. Erfindungsgemäß ist pro Zelle zumindestens ein Federelement vorgesehen. Das Federelement kann beispielsweise eine U- oder V-förmige Schleife in einem der Stege bzw. Schenkel jeder Zelle sein. Somit ist es mit der Erfindung möglich, einen Multizellularstent zu bilden, der eine Vielzahl von beispielsweise rautenförmigen oder zumindest rautenähnlichen Zellen aufweist. Die die Zellen bzw. Rauten umgrenzenden Schenkel bzw. Stege können hierbei die Federelemente aufweisen, welche im nichtgespreizten Zustand ausreichende Flexibilität über der Längsachse möglich machen.

Die Unteransprüche haben vorteilhafte Weiterbildungen der Erfindung zum Inhalt.

Weitere Einzelheiten, Merkmale und Vorteile der Erfindung ergeben sich aus nachfolgender Beschreibung von Ausführungsbeispielen anhand der Zeichnung. Es zeigt:
- Fig. 1a: eine schematisch stark vereinfachte Darstellung des Grundaufbaus eines erfindungsgemäßen Stents,
- Fig. 1b: eine Darstellung einer weiteren Ausführungsform einer Stegstruktur der Wand des Stents gemäß Fig. 1a,
- Fig. 2: eine Darstellung eine Ausführungsform des erfindungsgemässen Stents,
- Fig. 3: eine der Fig. 2 entsprechende Darstellung einer Abwandlung der Ausführungsform der Stegstruktur gemäß Fig. 2, und
- Fig. 4: eine Abwandlung der Ausführungsform von Fig. 3.

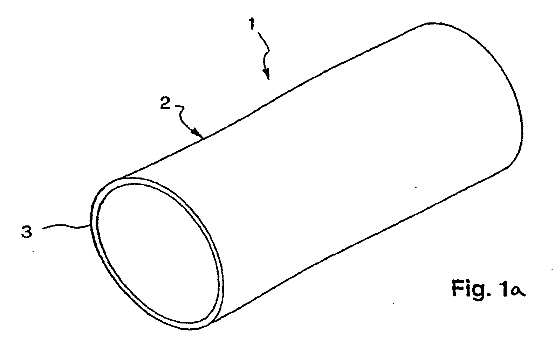

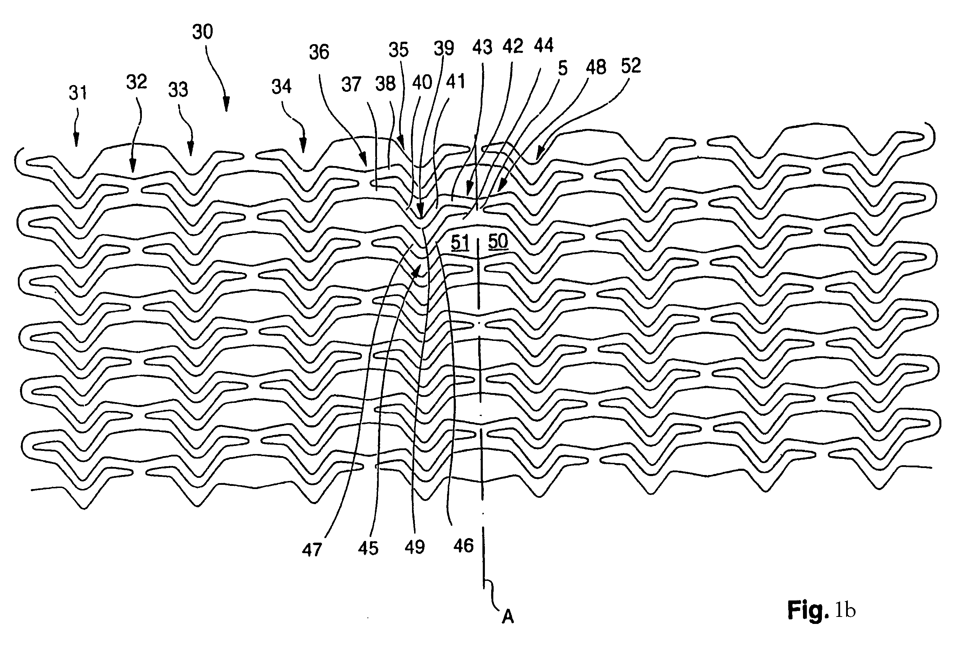

Fig. 1a zeigt den grundsätzlichen Aufbau eines erfindungsgemäßen Stents 1, der einen flexiblen, rohrförmigen Körper 2 mit einer Wand 3 aufweist, von der in Fig. 1 die Stirnansicht dargestellt ist.

In Fig. 1b ist eine Ausführungsform einer Stegstruktur 30 für einen Stent gemäß Fig. 1a dargestellt. Auch bei der Darstellung der Fig. 1b ist ein Ausschnitt der Wand des rohrförmigen Stegs in ebener Darstellung verdeutlicht. Der Stent mit dem Stegmuster gemäß Fig. 1b kann als ein Multizellularstent bezeichnet werden, der aus Rauten aufgebaut ist. Jede dieser Rauten weist in jedem Schenkel ein U- oder V-förmiges Federelement auf, welches im nicht gespreizten Zustand eine gewisse Flexibilität über der Längsachse garantiert.

Im einzelnen weist die Stegstruktur 30 eine Mehrzahl von nebeneinander angeordneten Stegmustern auf, von denen in Fig. 1b die Stegmuster 31, 33, 34, 35 und 52 mit entsprechenden Bezugsziffern gekennzeichnet sind. Diese Stegmuster bilden aufgrund der Rohrform des Stents radial verlaufende Wandstrukturen. Wie anhand des Beispieles der Stegmuster 31 und 33 in Fig. 1b verdeutlicht wurde, spannen diese mit einem Federelement 32 jeweils eine Zelle auf. Die Elemente 32 bilden hierbei, wie Fig. 1b verdeutlicht, integrale Bestandteile jeweils benachbarter Stegmuster. Anhand des Stegmusters 35 wird nachfolgend beispielhaft der Aufbau aller Stegmuster der Stegstruktur 30 erläutert.

Das Stegmuster 35 weist eine erste Schlaufe 36 auf, die zwei im spitzen Winkel zueinander angeordnete miteinander verbundene Schenkel 37, 38 aufweist. An die erste Schlaufe 36 schließt sich eine zweite Schlaufe 39 mit ebenfalls im spitzen Winkel zueinander angeordneten geraden Schenkeln 40, 41 an. An die Schlaufe 39 wiederum schließt sich eine dritte Schlaufe 42 an, die im spitzen Winkel zueinander angeordnete gerade Schenkel 43 und 44 aufweist. An die dritte Schlaufe 42 schließt sich eine vierte Schlaufe 45 mit wiederum geraden Schenkeln 46 und 47 an. Auch die Schenkel 46 und 47 der vierten Schlaufe 45 sind im spitzen Winkel zueinander angeordnet. Bei der in Fig. 1b dargestellten Ausführungsform bilden die Schenkel der jeweiligen Schlaufen 36, 39, 42 und 45 jeweils eine in etwa V-förmige Konfiguration. Grundsätzlich wäre auch eine U-förmige Konfiguration möglich. Wie Fig. 7 ferner verdeutlicht, wird ein komplettes Stegmuster natürlich durch eine immer wiederkehrende Abfolge von 4 Schlaufen in der zuvor beschriebenen Weise gebildet. Die Zahl der Schlaufen hängt von der jeweiligen Größe des Stents ab, so daß sich je nach Stentdurchmesser durch die Abfolge der Mehrzahl von vier Schlaufen geschlossene Ringe ergeben, die jeweils zusammen mit den Elementen 32 eine Zelle aufspannen.

Fig. 1b verdeutlicht ferner durch die Einzeichnung einer Achse A, daß das sich rechts an das zuvor beschriebene Stegmuster 35 anschließende Stegmuster 52 durch Spiegelung bzw. Umklappung des Stegmusters 35 um 180° ergibt. Somit ergibt sich für die gesamte Stegstruktur 30 stets eine Abfolge von jeweils an einer Achse A gespiegelten Stegmustern, wie dies zuvor am Beispiel der Stegmuster 35 und 52 beschrieben wurde.

Die Verbindung zwischen den Stegmustern 35 und 52 wird in Fig. 1b am Beispiel der Schlaufen 42 des Stegmusters 35 und 48 des Stegmusters 52 verdeutlicht. Es ergibt sich eine Verbindung im jeweiligen Scheitelpunkt S dieser Schlaufen 42 bzw. 48, wobei im Beispielsfalle die Scheitelpunkte S auf der Achse A liegen. Zu beiden Seiten der Achse A ergeben sich Zellen mit Teilräumen 50 und 51, die einen rautenähnlichen Gesamtraum bilden, der von den Schenkeln der zugeordneten Schlaufen begrenzt wird. Die Schlaufen 42 und 48 bilden somit ein weiteres Beispiel für ein Federelement entsprechend dem Element 32, das zuvor bereits erläutert wurde. In diesem Fall bilden die Elemente 32 aufgrund ihrer Ausbildung die den jeweiligen zellen zugeordneten Federelemente bzw. weisen derartige Federelemente auf.

Wie Fig. 1b schließlich verdeutlicht, weisen bei der darge-. stellten Ausführungsform alle Scheitelpunkte der sich gemäß der in Fig. 1b gewählten Darstellung nach oben öffnenden zweiten Schlaufen 39 in die gleiche Richtung, wobei zur Verdeutlichung der Scheitelpunkt 49 eingezeichnet ist. Im Beispielsfalle weisen aufgrund der in Fig. 1b gewählten Darstellung somit alle Scheitelpunkte nach unten.

Ferner weisen alle Scheitelpunkte der sich in Fig. 1b nach rechts öffnenden ersten Schlaufen 36 nach links, während die Scheitelpunkte der sich nach links öffnenden Schlaufen 42 nach rechts weisen. Jeweils umgekehrt ist es bei den gespiegelten Stegmustern, wie beispielsweise beim Stegmuster 52. Auch hier wird in Ergänzung zur schriftlichen Offenbarung zur Beschreibung und Offenbarung des Stegmusters 30 explizit auf die zeichnerische Darstellung der Fig. 1b verwiesen.

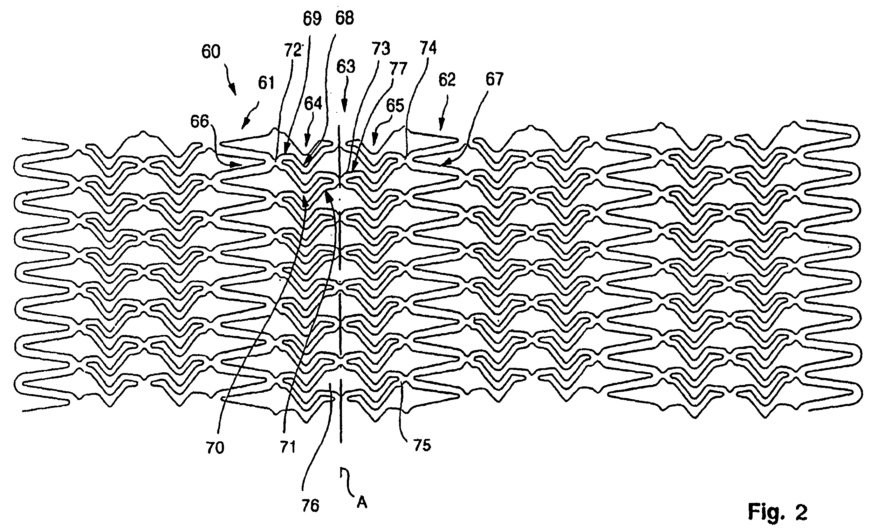

In Fig. 2 ist eine Ausführungsform einer Stegstruktur 60 für den Stent gemäß Fig. 1 dargestellt. Auch diese Ausführungsform entspricht in ihrer Darstellung der Fig. 8 einer planen Darstellung eines Teils der Wand des Stents. Diese weitere Ausführungsform kann als ein Stent beschrieben werden, der einzelne radial verlaufende Stegmuster (mäanderartig bzw. entsprechend einer Sinusschwingung verlaufend) aufweist, die über Rauten miteinander gekoppelt sind, die ihrerseits in jedem Schenkel ein U- oder alternativ V-förmiges Federelement aufweisen, welches im nicht gespreizten Zustand eine erforderliche Flexibilität über der Längsachse ermöglicht. Vorteilhafterweise können die ersten Stegmuster (Mäander) in ihrer Stegbreite stärker ausgelegt werden als die mit ihnen (noch zu beschreibenden) eine Zelle aufspannenden Elemente, so daß durch die ersten Stegmuster eine hohe radiale Kraft aufgenommen werden kann. Im einzelnen ist die Stegstruktur 60 gemäß Fig. 8 wie folgt aufgebaut:

Es ist eine Mehrzahl von Stegmustern vorgesehen, von denen in der Fig. 2 beispielhaft die Stegmuster 61 und 62 mit Bezugszeichen gekennzeichnet sind. Wie Fig. 2 verdeutlicht, sind die Stegmuster 61, 62 nebeneinander angeordnet und weisen im wesentlichen parallele Achsen auf. Die Stegmuster 61 und 62 können exakt einer Sinusschwingung entsprechen oder zumindest einen ähnlichen Verlauf haben, so daß sie auch als mäandrierende bzw. gewunden verlaufende Stegmuster bezeichnet werden können. Sie sind jeweils 180° außer Phase angeordnet, was erläutert am Beispiele der Stegmuster 61 und 62 bedeutet, daß einer sich nach rechts öffnenden Schlaufe des Stegmusters 61 eine sich nach links öffnende Schlaufe des Stegmusters 62 gegenüberliegt. Es versteht sich, daß auch bei dieser Ausführungsform die Stegmuster 61, 62 im tatsächlichen röhrchenförmigen Zustand des Stents Ringe der Wand des Stents bilden, wobei natürlich entsprechend der Länge des Stents eine entsprechende Anzahl von Stegmustern 61, 62 vorgesehen ist. Nachfolgend wird jedoch zur Beschreibung stets auf die Stegmuster 61, 62 beispielhaft Bezug genommen.

Diese Stegmuster 61, 62 spannen zusammen mit Elementen (Federelementen) 63 Zellen auf.

Das Element 63 ist bei der dargestellten Ausführungsform aus zwei Federelement- bzw. Stegmustern 64, 65 aufgebaut. Wie Fig. 2 verdeutlicht, entstehen die Federelement-Stegmuster 64, 65 durch Spiegelung bzw. Klappung an einer Symmetrieachse A. Die Federelement-Stegmuster 64, 65 weisen jeweils vier aneinander sich anschliessende Schlaufen 68 bis 71 auf, deren Aufbau geradlinige im spitzen Winkel zueinander angeordneten Schenkeln zeigt. Die Schlaufen 68 bis 71 sind jeweils einstückig miteinander verbunden, so daß die Mehrzahl dieser Schlaufen die Struktur der Federelement-Stegmuster ergibt, zu deren Offenbarung wieder explizit auf Fig. 2 Bezug genommen wird.

Fig. 2 zeigt ferner, daß das Stegmuster 61 zusammen mit Knotenpunkten jeweils an den Scheitelpunkten seiner Schlaufen und mit dem Element 63 eine Zelle aufspannt. Repräsentativ ist ein Knotenpunkt 72 eingezeichnet. Die Federelement-Stegmuster 64 und 65 sind über eine Mehrzahl von Knotenpunkten miteinander verbunden, was durch den Knotenpunkt 73 symbolisiert ist. Das nachfolgende Stegmuster 62 spannt ebenfalls mit einer Mehrzahl von Knotenpunkten jeweils im Scheitelpunkt seiner Schlaufen und mit dem Element 63 eine Zelle auf, wobei wiederum repräsentativ ein Knotenpunkt 74 eingezeichnet ist. Wie Fig. 2 verdeutlicht, ist der Knotenpunkt 72 der Scheitelpunkt einer Schlaufe 66 des Stegmusters 61 während der Knotenpunkt 74 der Scheitelpunkt einer Schlaufe 67 des Stegmusters 62 ist.

Wie auch im Falle der Ausführungsform der Fig. 1b weisen die Scheitelpunkte der Schlaufen 66 bis 67 der Federelement-Stegmuster jeweils in die gleiche Richtung. Insofern kann auf die Ausführungen zu Fig. 1b Bezug genommen werden, wobei zur Vereinfachung des Verständnisses repräsentativ ein Scheitelpunkt 75 einer Schlaufe des rechten Federelement-Stegmusters 65 eingezeichnet ist.

Zu ergänzen ist ferner, daß die Schenkel der Schlaufen der Federelement-Stegmuster 64, 65 jeweils Zellen 76 mit zugeordneten Federelementen begrenzen. Auch hier ist in Fig. 2 repräsentativ eine Kammer 76 mit diesem Bezugszeichen versehen.

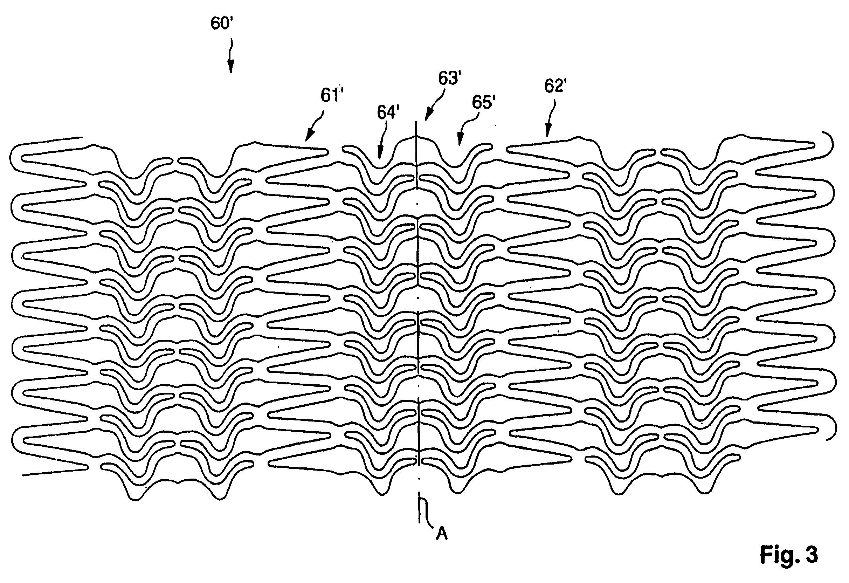

Fig. 3 entspricht im wesentlichen der Ausführungsform gemäß Fig. 2, so daß die Bezugsziffern der wesentlichen Elemente (einfach gestrichen) entsprechend der Ausführungsform gemäß Fig. 2 eingezeichnet sind. Die Fig. 3 verdeutlicht hierbei, daß das Element 63 bzw. dessen Schlaufen etwas mehr abgerundet als im Falle der Fig. 8 ausgebildet ist. Wie auch im Falle der vorhergehenden Ausführungsformen wird zur Offenbarung der Stegstruktur gemäß Fig. 3 explizit auf die Zeichnung Bezug genommen.

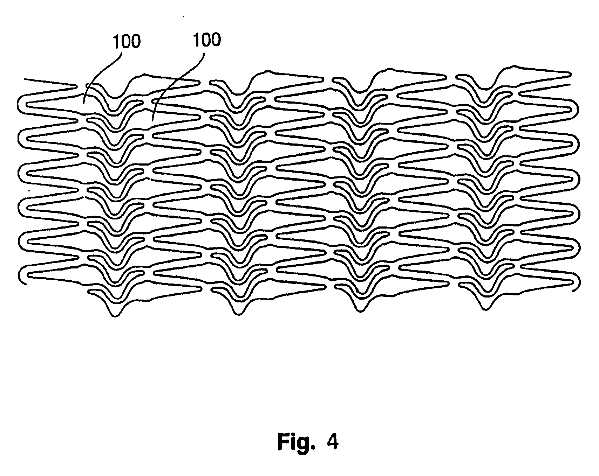

Schließlich zeigt Fig. 4 ein Fig. 3 ähnliches. Ausführungsbeispiel eines erfindungsgemäßen Stents. Bei diesem Ausführungsbeispiel werden ausschließlich gleichförmige Zellen aufgespannt, wobei lediglich zwei Zellen repräsentativ mit dem Bezugszeichen 100 versehen sind.

Im Gegensatz zu den Ausführungsbeispielen der Fig. 2 und 3, umschließen die Stege eine Zelle derart, daß jeweils ein Teil der Zelle aus geraden Stegen und ein zweiter Teil aus Stegen besteht, die jeweils ein Federelement aufweisen. Der linke Teil einer Zelle bildet jeweils den rechten Teil der links angrenzenden Zelle, der rechte Teil einer Zelle jeweils den linken Teil der rechts angrenzenden Zelle. Die rechts und links anschließenden Zellen sind jeweils um 180° gedreht, ansonsten aber deckungsgleich.

Fig. 4 beschreibt demnach einen Stent, welcher aus gleichförmigen Zellen aufgebaut ist, in der 2 D-Darstellung jeweils um 180° gedreht sind. Eine Zelle wird dabei immer von zwei geraden Schenkeln und zwei Schenkeln mit Federelementen aufgespannt. Die Federelemente in den Schenkeln ermöglichen eine hohe axiale Flexibilität im nicht expandierten Zustand.

Bezüglich der verschiedenen Komponenten, deren Ausgestaltung und Funktion dieses Ausführungsbeispiels wird auf obige Erläuterungen und Ausführungsformen verwiesen.

## Patentansprüche

1. Stent (1) mit einem rohrförmigen, flexiblen Körper (2), dessen Wand (3) eine Stegstruktur (60; 60') mäanderförmig verlaufender Stegmuster mit Scheitelpunkten (72;74) aufweist, die eine Vielzahl von aneinander angrenzenden Zellen (76; 100) aufweist, die von Stegen der Stegstruktur (60; 60') umgrenzt sind, wobei pro Zelle (76; 100) zumindest ein Federelement (63; 63') vorgesehen ist, und wobei die Federelemente (63; 63') als Federelement-Stegmuster (64, 65; 64', 65') ausgebildet sind, die jeweils vier sich aneinanderreihende Schlaufen (68 bis 71) aufweisen, wobei die Schlaufen (68 bis 71) einstückig miteinander verbunden sind, **dadurch gekennzeichnet, dass** die Stegstruktur (60, 60') jeweils an ihren Scheitelpunkten (72, 74) mit dem Scheitelpunkt einer der Schlaufen des benachbarten Federelement-Stegmusters verbunden ist, und dass die Stegstruktur (60, 60') in ihrer Stegbreite stärker als die der Federelemente (63, 63') ausgelegt ist.

2. Stent nach Anspruch 1, **gekennzeichnet durch** zumindest ein Federelement (63; 63') mit zwei gegenüber einer Längsachse (A) um 180° geklappten Federelement-Stegmustern (64, 65; 64', 65').

3. Stent nach Anspruch 1 oder 2, **dadurch gekennzeichnet, daß** die Schlaufen (68-71) jeweils in etwa V-förmig ausgebildet sind.

4. Stent einem der Ansprüche 1 bis 3, **dadurch gekennzeichnet, daß** die Federelement-Stegmuster (64, 65; 64', 65') jeweils über mit ihren Scheitelpunkten (73) aneinander angrenzende Schlaufen (71, 77) miteinander eine Zelle aufspannen.

## Claims

1. Stent (1) with a tubular flexible body (2) whose wall (3) exhibits a strut structure (60; 60') of meandering strut pattern with vertices (72; 74), which exhibits a multitude of cells (76; 100) that adjoin one another, which are enclosed by struts of said strut structure (60; 60'), whereby at least one spring element (63; 63'), is provided per said cell (76; 100), and whereby said spring elements (63; 63') are formed as spring element - strut patterns (64, 65, 64', 65'), which each exhibit four loops (68 to 71) joined up end to end, whereby said loops are singly linked together, **characterised in that** said strut structure (60; 60') is linked at each of its vertices (72, 74) to the vertex of one of the loops of the adjoining spring element - strut pattern, and that said strut structure (60; 60') is designed with a greater strut thickness than that of said spring element (63; 63').

2. Stent according to claim 1, **characterised by** at least one said spring element (63; 63') with two said spring element - strut patterns (64, 65, 64', 65') folded through 180°, with respect to a longitudinal axis (A).

3. Stent according to claim 1 or 2, **characterised in that** said loops are each formed roughly in a v-shape.

4. Stent according to one of claims 1 to 3, **characterised in that** said spring element - strut patterns (64, 65, 64', 65') each jointly span a cell via loops (71, 77) that adjoin one another via their vertices (73).

## Revendications

1. Endoprothèse coronaire (1) avec un corps (2) tubulaire flexible, dont la paroi (3) comporte une structure à barrettes (60 ; 60') avec un modèle de barrettes s'étendant sous forme de méandres avec des sommets (72 ; 74), qui comporte une pluralité de cellules (76 ; 100) adjacentes qui sont délimitées par des barrettes de la structure à barrettes (60 ; 60'), au moins un élément à ressort (63 ; 63') étant prévu pour chaque cellule (76 ; 100) et les éléments à ressort (63 ; 63') étant conçus sous forme de modèle de barrettes et éléments de ressort (64, 65 ; 64', 65') qui comportent chacun quatre boucles (68 à 71) alignées, les boucles (68 à 71) étant assemblées d'un seul tenant entre elles, **caractérisée en ce que** la structure à barrettes (60 ; 60') est reliée au niveau de chacun de ses sommets (72, 74) avec le sommet de l'une des boucles du modèle de barrettes et éléments à ressort voisin, et **en ce que** la structure à barrettes (60 ; 60') a des barrettes avec une largeur supérieure à celle des éléments à ressort (63 ; 63').

2. Endoprothèse coronaire selon la revendication 1, **caractérisée par** au moins un élément à ressort (63 ; 63') avec deux modèles de barrettes et éléments à ressort (64, 65 ; 64', 65') rabattus sur 180° par rapport à un axe longitudinal (A).

3. Endoprothèse coronaire selon la revendication 1 ou 2, **caractérisée en ce que** les boucles (68-71) sont conçues chacune pratiquement en forme de V.

4. Endoprothèse coronaire selon l'une quelconque des revendications 1 à 3, **caractérisée en ce que** les modèles de barrettes et éléments à ressort (64, 65 ; 64', 65') déploient chacun entre eux une cellule par l'intermédiaire de boucles (71, 77) adjacentes par leurs sommets (73).
